# EUROPEAN PATENT APPLICATION

(11) **EP 2 261 298 A1**
(43) Date of publication of application: **15.12.2010**
(21) Application number: 09162454.4
(22) Date of filing: 10.06.2009
(51) Int. Cl.: C09K 8/584

(54) **Method for enhanced hydrocarbon recovery**

(71) Applicant: Shell Internationale Research Maatschappij B.V., 2596 HR Den Haag (NL)
(72) Inventor: Anderson, Steven Ray, 2288 GS Rijswijk (NL); Moene, Robert, 1031 HW Amsterdam (NL); Wood, Paul, 1031 HW Amsterdam (NL)
(74) Representative: Matthezing, Robert Maarten

(57) **Abstract**

The present invention provides method of treating a hydrocarbon containing formation, said process comprising the steps of
a) forming a hydrocarbon recovery composition;
b) providing said composition to at least a portion of the hydrocarbon containing formation; and
c) allowing the composition to interact with the hydrocarbons in the hydrocarbon containing formation,

wherein step a) is carried out in situ at the location where the composition is provided to at least a portion of the hydrocarbon containing formation in step b).

## Description

### Field of the Invention

The present invention relates to methods for recovery of hydrocarbons from hydrocarbon formations. More particularly, embodiments described herein relate to methods of enhanced hydrocarbons recovery.

### Background of the Invention

Hydrocarbons may be recovered from hydrocarbon containing formations by penetrating the formation with one or more wells. Hydrocarbons may flow to the surface through the wells. Conditions (e.g. permeability, hydrocarbon concentration, porosity, temperature, pressure) of the hydrocarbon containing formation may affect the economic viability of hydrocarbon production from the hydrocarbon containing formation. A hydrocarbon containing formation may have natural energy (e.g. gas, water) to aid in mobilizing hydrocarbons to the surface of the hydrocarbon containing formation. Natural energy may be in the form of water. Water may exert pressure to mobilize hydrocarbons to one or more production wells. Gas may be present in the hydrocarbon containing formation at sufficient pressures to mobilize hydrocarbons to one or more production wells. The natural energy source may become depleted over time. Supplemental recovery processes may be used to continue recovery of hydrocarbons from the hydrocarbon containing formation. Examples of supplemental processes include water flooding, polymer flooding, alkali flooding, thermal processes, solution flooding or combinations thereof.

Compositions and methods suitable for enhanced hydrocarbons recovery utilizing surfactant type molecules, such as alkoxylated alcohols, carboxylates, sulfonates or sulfates of alcohols or alkoxylated alcohols and derivatives thereof are known. Such materials are generally produced in large central chemical plants and then shipped great distances, often in highly diluted form. This shipping can incur a high level of cost both monetary and in terms of energy usage and CO₂ emissions.

It is known that different compositions will be required in order to promote hydrocarbons recovery in a specific hydrocarbon containing formation. Thus, it may also be difficult to supply suitable quantities of a composition tailored to a specific hydrocarbon containing formation from a central chemical plant in a timely manner.

### Summary of the Invention

According to the present invention, there is provided a method of treating a hydrocarbon containing formation, said process comprising the steps of
a) forming a hydrocarbon recovery composition;
b) providing said composition to at least a portion of the hydrocarbon containing formation; and
c) allowing the composition to interact with the hydrocarbons in the hydrocarbon containing formation,
wherein step a) is carried out in situ at the location where the composition is provided to at least a portion of the hydrocarbon containing formation in step b).

### Brief Description of the Drawings

FIG. 1 depicts an embodiment of treating a hydrocarbon containing formation; and
FIG. 2 depicts an embodiment of treating a hydrocarbon containing formation.

### Detailed Description of the Invention

Hydrocarbons may be produced from hydrocarbon formations through wells penetrating a hydrocarbon containing formation. "Hydrocarbons" are generally defined as molecules formed primarily of carbon and hydrogen atoms such as oil and natural gas. Hydrocarbons may also include other elements, such as, but not limited to, halogens, metallic elements, nitrogen, oxygen and/or sulfur. Hydrocarbons derived from a hydrocarbon formation may include, but are not limited to, kerogen, bitumen, pyrobitumen, asphaltenes, oils or combinations thereof. Hydrocarbons may be located within or adjacent to mineral matrices within the earth. Matrices may include, but are not limited to, sedimentary rock, sands, silicilytes, carbonates, diatomites and other porous media.

A "formation" includes one or more hydrocarbon containing layers, one or more non-hydrocarbon layers, an overburden and/or an underburden. An "overburden" and/or an "underburden includes one or more different types of impermeable materials. For example, overburden/underburden may include rock, shale, mudstone, or wet/tight carbonate (i.e., an impermeable carbonate without hydrocarbons). For example, an underburden may contain shale or mudstone. In some cases, the overburden/underburden may be somewhat permeable. For example, an underburden may be composed of a permeable mineral such as sandstone or limestone. In some embodiments, at least a portion of a hydrocarbon containing formation may exist at less than or more than 1000 feet below the earth's surface.

Properties of a hydrocarbon containing formation may affect how hydrocarbons flow through an underburden/overburden to one or more production wells. Properties include, but are not limited to, porosity, permeability, pore size distribution, surface area, salinity or temperature of formation. Overburden/underburden properties in combination with hydrocarbon properties, such as, capillary pressure (static) characteristics and relative permeability (flow) characteristics may effect mobilization of hydrocarbons through the hydrocarbon containing formation.

Permeability of a hydrocarbon containing formation may vary depending on the formation composition. A relatively permeable formation may include heavy hydrocarbons entrained in, for example, sand or carbonate. "Relatively permeable," as used herein, refers to formations or portions thereof, that have an average permeability of 10 millidarcy or more. "Relatively low permeability" as used herein, refers to formations or portions thereof that have an average permeability of less than about 10 millidarcy. One darcy is equal to about 0.99 square micrometers. An impermeable portion of a formation generally has a permeability of less than about 0.1 millidarcy. In some cases, a portion or all of a hydrocarbon portion of a relatively permeable formation may include predominantly heavy hydrocarbons and/or tar with no supporting mineral grain framework and only floating (or no) mineral matter (e.g. asphalt lakes).

Fluids (e.g. gas, water, hydrocarbons or combinations thereof) of different densities may exist in a hydrocarbon containing formation. A mixture of fluids in the hydrocarbon containing formation may form layers between an underburden and an overburden according to fluid density. Gas may form a top layer, hydrocarbons may form a middle layer and water may form a bottom layer in the hydrocarbon containing formation. The fluids may be present in the hydrocarbon containing formation in various amounts. Interactions between the fluids in the formation may create interfaces or boundaries between the fluids. Interfaces or boundaries between the fluids and the formation may be created through interactions between the fluids and the formation. Typically, gases do not form boundaries with other fluids in a hydrocarbon containing formation. In an embodiment, a first boundary may form between a water layer and underburden. A second boundary may form between a water layer and a hydrocarbon layer. A third boundary may form between hydrocarbons of different densities in a hydrocarbon containing formation. Multiple fluids with multiple boundaries may be present in a hydrocarbon containing formation, in some embodiments. It should be understood that many combinations of boundaries between fluids and between fluids and the overburden/underburden may be present in a hydrocarbon containing formation.

Production of fluids may perturb the interaction between fluids and between fluids and the overburden/underburden. As fluids are removed from the hydrocarbon containing formation, the different fluid layers may mix and form mixed fluid layers. The mixed fluids may have different interactions at the fluid boundaries. Depending on the interactions at the boundaries of the mixed fluids, production of hydrocarbons may become difficult. Quantification of the interactions (e.g. energy level) at the interface of the fluids and/or fluids and overburden/underburden may be useful to predict mobilization of hydrocarbons through the hydrocarbon containing formation.

Quantification of energy required for interactions (e.g. mixing) between fluids within a formation at an interface may be difficult to measure. Quantification of energy levels at an interface between fluids may be determined by generally known techniques (e.g. spinning drop tensiometer). Interaction energy requirements at an interface may be referred to as interfacial tension. "Interfacial tension" as used herein, refers to a surface free energy that exists between two or more fluids that exhibit a boundary. A high interfacial tension value (e.g. greater than about 10 dynes/cm) may indicate the inability of one fluid to mix with a second fluid to form a fluid emulsion. As used herein, an "emulsion" refers to a dispersion of one immiscible fluid into a second fluid by addition of a composition that reduces the interfacial tension between the fluids to achieve stability. The inability of the fluids to mix may be due to high surface interaction energy between the two fluids. Low interfacial tension values (e.g. less than about 1 dyne/cm) may indicate less surface interaction between the two immiscible fluids. Less surface interaction energy between two immiscible fluids may result in the mixing of the two fluids to form an emulsion. Fluids with low interfacial tension values may be mobilized to a well bore due to reduced capillary forces and subsequently produced from a hydrocarbon containing formation.

Fluids in a hydrocarbon containing formation may wet (e.g. adhere to an overburden/underburden or spread onto an overburden/underburden in a hydrocarbon containing formation). As used herein, "wettability" refers to the preference of a fluid to spread on or adhere to a solid surface in a formation in the presence of other fluids. Methods to determine wettability of a hydrocarbon formation are described by Craig, Jr. in "The Reservoir Engineering Aspects of Waterflooding", 1971 Monograph Volume 3, Society of Petroleum Engineers. In an embodiment, hydrocarbons may adhere to sandstone in the presence of gas or water. An overburden/underburden that is substantially coated by hydrocarbons may be referred to as "oil wet". An overburden/underburden may be oil wet due to the presence of polar and/or heavy hydrocarbons (e.g. asphaltenes) in the hydrocarbon containing formation. Formation composition (e.g. silica, carbonate or clay) may determine the amount of adsorption of hydrocarbons on the surface of an overburden/underburden. In some embodiments, a porous and/or permeable formation may allow hydrocarbons to more easily wet the overburden/underburden. A substantially oil wet overburden/underburden may inhibit hydrocarbon production from the hydrocarbon containing formation. In certain embodiments, an oil wet portion of a hydrocarbon containing formation may be located at less than or more than 1000 feet below the earth's surface.

A hydrocarbon formation may include water. Water may interact with the surface of the underburden. As used herein, "water wet" refers to the formation of a coat of water on the surface of the overburden/underburden. A water wet overburden/underburden may enhance hydrocarbon production from the formation by preventing hydrocarbons from wetting the overburden/underburden. In certain embodiments, a water wet portion of a hydrocarbon containing formation may include minor amounts of polar and/or heavy hydrocarbons.

Water in a hydrocarbon containing formation may contain minerals (e.g. minerals containing barium, calcium, or magnesium) and mineral salts (e.g. sodium chloride, potassium chloride, magnesium chloride). Water salinity and/or water hardness of water in a formation may affect recovery of hydrocarbons in a hydrocarbon containing formation. As used herein "salinity" refers to an amount of dissolved solids in water. "Water hardness," as used herein, refers to a concentration of divalent ions (e.g. calcium, magnesium) in the water. Water salinity and hardness may be determined by generally known methods (e.g. conductivity, titration). As used herein, "high salinity water" refers to water that has greater than about 30,000 ppm total dissolved solids based on sodium chloride. As water salinity increases in a hydrocarbon containing formation, interfacial tensions between hydrocarbons and water may be increased and the fluids may become more difficult to produce.

Low salinity water in a hydrocarbon containing formation may enhance hydrocarbon production from a hydrocarbon containing formation. Hydrocarbons and low salinity water may form a well dispersed emulsion due to a low interfacial tension between the low salinity water and the hydrocarbons. Production of a flowable emulsion (e.g. hydrocarbons/water mixture) from a hydrocarbon containing formation may be more economically viable to a producer. As used herein, "low salinity water" refers to water salinity in a hydrocarbon containing formation that is less than about 20,000 parts per million (ppm) total dissolved solids based on sodium chloride. In some embodiments, hydrocarbon containing formations may include water with a salinity of less than about 13,000 ppm. In certain embodiments, hydrocarbon containing formations may include water with a salinity ranging from about 3,000 ppm to about 10,000 ppm. In other embodiments, salinity of the water in hydrocarbon containing formations may range from about 5,000 ppm to about 8,000 ppm.

A hydrocarbon containing formation may be selected for treatment based on factors such as, but not limited to, thickness of hydrocarbon containing layers within the formation, assessed liquid production content, location of the formation, salinity content of the formation, temperature of the formation, and depth of hydrocarbon containing layers. Initially, natural formation pressure and temperature may be sufficient to cause hydrocarbons to flow into well bores and out to the surface. Temperatures in a hydrocarbon containing formation may range from about 0 °C to about 300 °C. As hydrocarbons are produced from a hydrocarbon containing formation, pressures and/or temperatures within the formation may decline. Various forms of artificial lift (e.g. pumps, gas injection) and/or heating may be employed to continue to produce hydrocarbons from the hydrocarbon containing formation. Production of desired hydrocarbons from the hydrocarbon containing formation may become uneconomical as hydrocarbons are depleted from the formation.

Mobilization of residual hydrocarbons retained in a hydrocarbon containing formation may be difficult due to viscosity of the hydrocarbons and capillary effects of fluids in pores of the hydrocarbon containing formation. As used herein "capillary forces" refers to attractive forces between fluids and at least a portion of the hydrocarbon containing formation. In an embodiment, capillary forces may be overcome by increasing the pressures within a hydrocarbon containing formation. In other embodiments, capillary forces may be overcome by reducing the interfacial tension between fluids in a hydrocarbon containing formation. The ability to reduce the capillary forces in a hydrocarbon containing formation may depend on a number of factors, including, but not limited to, the temperature of the hydrocarbon containing formation, the salinity of water in the hydrocarbon containing formation, and the composition of the hydrocarbons in the hydrocarbon containing formation.

As production rates decrease, additional methods may be employed to make a hydrocarbon containing formation more economically viable. Methods may include adding sources of water (e.g. brine, steam), gases, polymers, monomers or any combinations thereof to the hydrocarbon formation to increase mobilization of hydrocarbons.

In an embodiment, a hydrocarbon containing formation may be treated with a flood of water. A waterflood may include injecting water into a portion of a hydrocarbon containing formation through injections wells. Flooding of at least a portion of the formation may water wet a portion of the hydrocarbon containing formation. The water wet portion of the hydrocarbon containing formation may be pressurized by known methods and a water/hydrocarbon mixture may be collected using one or more production wells. The water layer, however, may not mix with the hydrocarbon layer efficiently. Poor mixing efficiency may be due to a high interfacial tension between the water and hydrocarbons.

Production from a hydrocarbon containing formation may be enhanced by treating the hydrocarbon containing formation with a polymer and/or monomer that may mobilize hydrocarbons to one or more production wells. The polymer and/or monomer may reduce the mobility of the water phase in pores of the hydrocarbon containing formation. The reduction of water mobility may allow the hydrocarbons to be more easily mobilized through the hydrocarbon containing formation. Polymers include, but are not limited to, polyacrylamides, partially hydrolyzed polyacrylamide, polyacrylates, ethylenic copolymers, biopolymers, carboxymethylcellulose, polyvinyl alcohol, polystyrene sulfonates, polyvinylpyrrolidone, AMPS (2-acrylamide-2-methyl propane sulfonate) or combinations thereof. Examples of ethylenic copolymers include copolymers of acrylic acid and acrylamide, acrylic acid and lauryl acrylate, lauryl acrylate and acrylamide. Examples of biopolymers include xanthan gum and guar gum.

In some embodiments, polymers may be crosslinked in situ in a hydrocarbon containing formation. In other embodiments, polymers may be generated in situ in a hydrocarbon containing formation. Polymers and polymer preparations for use in oil recovery are described in U.S. Patent No. 6,427,268 to Zhang et al., entitled "Method For Making Hydrophobically Associative Polymers, Methods of Use and Compositions;" U.S. Patent No. 6,439,308 to Wang, entitled "Foam Drive Method;" U.S. Patent No. 5,654,261 to Smith, entitled, "Permeability Modifying Composition For Use In Oil Recovery;" U.S. Patent No. 5,284,206 to Surles et al., entitled "Formation Treating;" U.S. Patent 5,199,490 to Surles et al., entitled "Formation Treating" and U.S. Patent No. 5,103,909 to Morgenthaler et al., entitled "Profile Control In Enhanced Oil Recovery.

In the present invention, a hydrocarbon recovery composition is provided to the hydrocarbon containing formation as well as, or instead of, some of the methods to make a hydrocarbon containing formation more economically viable described above. The formation of the hydrocarbon recovery composition, i.e. step a) of the process of the present invention, is carried out in situ at the location where the composition is provided to at least a portion of the hydrocarbon containing formation. That is, at least the last chemical transformation in the synthesis of a component of the hydrocarbon recovery composition is carried out in a reactor set-up at the site on the Earth's surface at which the composition is provided to at least a portion of the hydrocarbon containing formation. Step a) of the process of the present invention may also include combination of said component of the hydrocarbon recovery composition with other components and/or dilution of the component(s) in order to produce the hydrocarbon recovery composition which is provided to at least a portion of the hydrocarbon containing formation.

The skilled person will understand herein that the phrase 'at the site' does not limit the location to the precise geographical point at which the composition is provided to at least a portion of the hydrocarbon containing formation, but to an area within a limited radius of this point. Preferably, said radius is no more than 50 miles, more preferably no more than 40 miles, even more preferably no more than 30 miles, still more preferably no more than 20 miles, yet more preferably no more than 10 miles, most preferably no more than 5 miles from the point at which the composition is provided to at least a portion of the hydrocarbon containing formation.

Suitable chemical transformations that fall under step a) of the process of the present invention include, but are not limited to, the alkoxylation of alcohols in order to make suitable alkoxylated alcohols; the sulfonation of olefins, alcohols or their alkoxylated derivatives in order to provide suitable sulfonate compounds; sulfation of alcohols or alkoxylated alcohols to provide suitable sulfates; carboxylation of alcohols or their alkoxylated derivatives in order to make suitable carboxylates; or sulfation of olefins followed by reaction with an alcohol, alcohol alkoxylate of other nucleophile.

In a preferred embodiment of the present invention, step a) of the process comprises sulfonation of an olefin, an alcohol or an alkoxylated alcohol in order to produce a suitable sulfonate hydrocarbon recovery composition.

Any suitable sulfonation process is applicable in the present invention. In one preferred embodiment a SO₃lair process based on sulfur burning is used.

The sulfonation may be carried out batchwise, semi-continuously or continuously, preferably continuously.

Sulfonation and subsequent treatment to form an surfactant sulfonate may be conveniently carried out in accordance with the methods described in US 4393937 A, EP 0351928 A1 and EP 0482687 A1.

Examples of sulfonating agents that may be conveniently used include not only sulphur trioxide (SO₃), but also any compounds or complexes which contain or yield SO₃. However, sulfur trioxide per se is particularly preferred for use as a sulfonating agent.

Preferably, the sulfonation reaction takes place between the one or more sulfonate precursor (such as monoolefins, alkyl benzenes, branched alkyl benzenes or alcohol alkoxylates) and a sulfonating agent, for example in a film reactor, in a mol ratio of sulfonating agent to precursor in the range of from 2.0 to 0.8, more preferably in the range of from 1.3 to 0.95, while cooling the reactor with a cooling means having a temperature between 10 and 70 °C and treating the reaction product to form the sulfonate or sulfate surfactant.

In one embodiment of the present invention, the sulfonation may be followed by neutralisation by reaction with a base, preferably an alkali or alkaline earth metal hydroxide, oxide or carbonate, followed by a subsequent after-treatment step, i.e. a so-called hydrolysis step.

The neutralisation may be preferably carried out continuously and at a temperature in the range of from 20 to 90 °C, more preferably in the range of from 30 to 70 °C. Hydrolysis is preferably carried out continuously and at a higher temperature than the neutralisation step, for example, up to and including 200 °C.

Sulfonates suitable for use in a hydrocarbon recovery composition are usually transported in diluted form, e.g. as a 20wt% active material solution in water. Such dilution is not required for the olefins, alcohols or alkoxylated alcohols from which they are formed. Thus, by applying the process of the present invention large energy and transport costs are saved.

The hydrocarbon recovery composition used in the process of the present invention may also comprise other components including, but not limited to, water, low molecular weight alcohols, organic solvents, alkyl sulfonates, aryl sulfonates, brine or combinations thereof. Low molecular weight alcohols include, but are not limited to, methanol, ethanol, propanol, isopropyl alcohol, *tert*-butyl alcohol, *sec*-butyl alcohol, butyl alcohol, *tert*-amyl alcohol or combinations thereof. Organic solvents include, but are not limited to, methyl ethyl ketone, acetone, lower alkyl cellosolves, lower alkyl carbitols or combinations thereof.

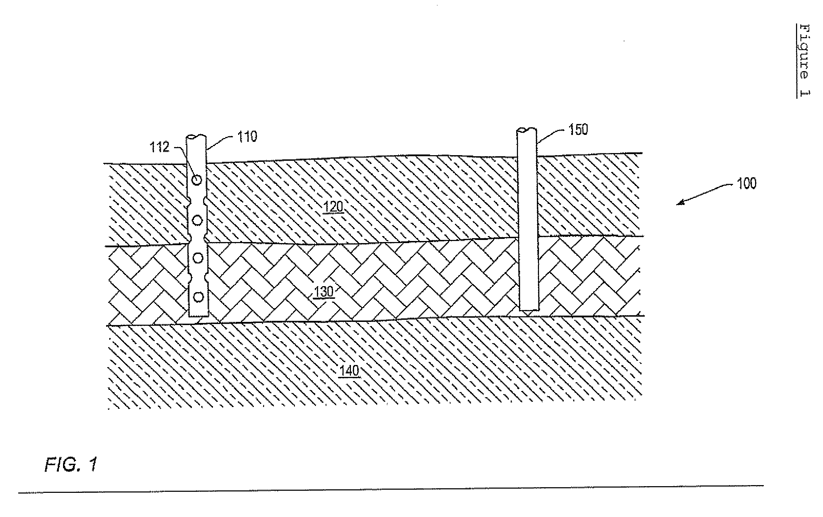

In an embodiment of the present invention after forming the hydrocarbon recovery composition, it may be provided (e.g., injected) into hydrocarbon containing formation 100 through injection well 110 as depicted in FIG. 1. Hydrocarbon formation 100 may include overburden 120, hydrocarbon layer 130, and underburden 140. Injection well 110 may include openings 112 that allow fluids to flow through hydrocarbon containing formation 100 at various depth levels. In certain embodiments, hydrocarbon layer 130 may be less than 1000 feet below earth's surface. In some embodiments, underburden 140 of hydrocarbon containing formation 100 may be oil wet. Low salinity water may be present in hydrocarbon containing formation 100, in other embodiments.

The hydrocarbon recovery composition may be provided to the formation in an amount based on hydrocarbons present in a hydrocarbon containing formation. The amount of hydrocarbon recovery composition, however, may be too small to be accurately delivered to the hydrocarbon containing formation using known delivery techniques (e.g., pumps). To facilitate delivery of small amounts of the hydrocarbon recovery composition to the hydrocarbon containing formation, the hydrocarbon recovery composition may be combined with water and/or brine to produce an injectable fluid. An amount of a hydrocarbon recovery composition injected into hydrocarbon containing formation 100 may be less than 0.5 wt.% of the total weight of the injectable fluid. In certain embodiments, an amount of a hydrocarbon recovery composition provided to a hydrocarbon containing formation may be less than 0.3 wt.% of the total weight of injectable fluid. In some embodiments, an amount of a hydrocarbon recovery composition provided to a hydrocarbon containing formation may be less than 0.1 wt.% of the total weight of injectable fluid. In other embodiments, an amount of a hydrocarbon recovery composition provided to a hydrocarbon containing formation may be less than 0.05 wt.% of the total weight of injectable fluid.

To facilitate delivery of an amount of the hydrocarbon recovery composition to the hydrocarbon containing formation, the hydrocarbon composition may be combined with water or brine to produce an injectable fluid. Less than about 0.5 wt% of the hydrocarbon recovery composition, based on the total weight of injectable fluid, may be injected into hydrocarbon containing formation 100 through injection well 110. In certain embodiments, the concentration of the hydrocarbon recovery composition injected through injection well 110 may be less than 0.3 wt.%, based on the total weight of injectable fluid. In some embodiments, the concentration of the hydrocarbon recovery composition may be less 0.1 wt.% based on the total weight of injectable fluid. In other embodiments, the concentration of the hydrocarbon recovery composition may be less 0.05 wt.% based on the total weight of injectable fluid.

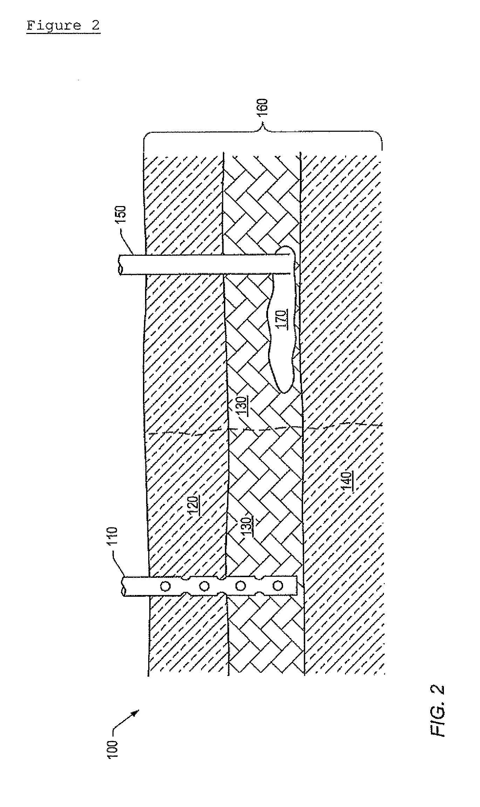

In some embodiments, a hydrocarbon recovery composition may be added to a portion of a hydrocarbon containing formation 100 that has an average temperature of from 0 to 150°C. In some embodiments, a hydrocarbon recovery composition may be combined with at least a portion of a hydrocarbon removal fluid (e.g. water, polymer solutions) to produce an injectable fluid. Less than about 0.5 wt% of the hydrocarbon recovery composition, based on the total weight of injectable fluid, may be injected into hydrocarbon containing formation 100 through injection well 110 as depicted in FIG. 2. In certain embodiments, a concentration of the hydrocarbon recovery composition injected through injection well 110 may be less than 0.3 wt.%, based on the total weight of injectable fluid. In some embodiments, less than 0.1 wt.% of the hydrocarbon recovery composition, based on the total weight of injectable fluid, may be injected through injection well 110 into hydrocarbon containing formation 100. In other embodiments, less than 0.05 wt.% of the hydrocarbon recovery composition, based on the total weight of injectable fluid, may be injected through injection well 110 into hydrocarbon containing formation 100.

In some embodiments, a hydrocarbon recovery composition may include an inorganic salt (e.g. sodium carbonate (Na₂CO₃), sodium chloride (NaCl), or calcium chloride (CaCl₂)). The addition of the inorganic salt may help the hydrocarbon recovery composition disperse throughout a hydrocarbon/water mixture. The enhanced dispersion of the hydrocarbon recovery composition may decrease the interactions between the hydrocarbon and water interface. The decreased interaction may lower the interfacial tension of the mixture and provide a fluid that is more mobile.

As well as a reduction in energy usage and transport costs, the present invention provides a simple process which allows tailoring of a hydrocarbon recovery composition to the specific hydrocarbon containing formation to which it is to be provided. The relative small scale of the process also allows the construction of any required reactor unit off-site. Such reactor units may be of a size such that it would prove viable to move them to a different site after they are no longer required at the first site. Further, it is likely that raw materials, such as sulfur or CO₂, may be available on site.

## Claims

1. A method of treating a hydrocarbon containing formation, said process comprising the steps of
a) forming a hydrocarbon recovery composition;
b) providing said composition to at least a portion of the hydrocarbon containing formation; and
c) allowing the composition to interact with the hydrocarbons in the hydrocarbon containing formation,
wherein step a) is carried out in situ at the location where the composition is provided to at least a portion of the hydrocarbon containing formation in step b).

2. A method as claimed in claim 1, wherein step a) comprises sulfonation of an olefin, an alcohol or an alkoxylated alcohol to provide a sulfonate component of the hydrocarbon recovery composition.

3. A method as claimed in claim 2, wherein sulfonation is carried out by a SO₃/air process based on sulfur burning.

4. A method as claimed in any one of claims 1 to 3, wherein step a) also comprises addition of components selected from the group comprising low molecular weight alcohols, organic solvents, alkyl sulfonates, aryl sulfonates or combinations thereof.

5. A method as claimed in any one of claims 1 to 4, wherein step a) also comprises the addition of water and/or brine to produce an injectable fluid.
